# EUROPEAN PATENT APPLICATION

(11) **EP 4 494 620 A1**
(43) Date of publication of application: **22.01.2025**
(21) Application number: 23826988.0
(22) Date of filing: 07.06.2023
(51) Int. Cl.: A61F 13/49, A61F 13/496, A61F 13/51, A61F 13/551

(54) **PANT-TYPE ABSORBENT ARTICLE**

(30) Priority: 20.06.2022 JP 2022099149
(71) Applicant: Unicharm Corporation, Ehime 799-0111 (JP)
(72) Inventor: SHIMAZU, Takeshi, Kanonji-shi, Kagawa 769-1602 (JP); TANAKA, Suguru, Kanonji-shi, Kagawa 769-1602 (JP); ISHII, Yudai, Kanonji-shi, Kagawa 769-1602 (JP)
(74) Representative: Dolleymores
(86) International application number: PCT/JP2023/021197
(87) International publication number: WO 2023/248801

(57) **Abstract**

Provided is a pant-type absorbent article (1) comprising: an absorbent body (10) including an absorbent core (11); and a torso member (20). The torso member (20) has, at least on one side thereof in the front-back direction, first elastic members (E1, 25) that are stretchable in the left-right direction and disposed in positions not overlapping the absorbent core (11) in the up-down direction, and second elastic members (E2, 27) that are stretchable in the left-right direction and disposed in positions overlapping the absorbent core (11) in the up-down direction. In an area where the torso member (20) in a stretched state and the absorbent core (11) overlap in the left-right direction, the total length of attachment means (50) for attaching the first elastic members (E1, 25) to the torso member (20) is shorter than the total length of attachment means (70) for attaching the second elastic members (E2, 27) to the torso member (20).

## Description

### FIELD

The present invention relates to an underpants-shaped absorbent article.

### BACKGROUND

Conventionally, there are known underpants-shaped absorbent articles in which a waist member has a plurality of elastic members that have the stretchability in the lateral direction. For example, Patent Document 1 discloses an underpants-shaped diaper in which a hot-melt adhesive is applied to the outer surfaces of elastic stretch members 12C, which are sandwiched between an outer layer 12S and an inner layer 12H that constitute an exterior sheet 12 (corresponding to the waist member), and the two layers 12S and 12H are bonded together with the adhesive and the elastic stretch members 12C.

### [CITATION LIST]

### [PATENT LITERATURE]

[Patent Document 1] Japanese Patent Application Publication No. 2012-61348

### SUMMARY

### [TECHNICAL PROBLEM]

In the underpants-shaped diaper of Patent Document 1, each of the multiple elastic stretch members 12Cs is attached to the waist member using the hot-melt adhesive that is evenly applied. That is, elastic members are attached to the waist member by uniform attachment means. However, if the lateral length of the attachment means is short in a portion that overlaps the absorbent core in the thickness direction, the portion where an elastic member is fixed is short and this makes the diaper more likely to stretch. Therefore, it becomes less likely to pull up the absorbent core when pulling the waist member and the absorbent core is less likely to come close contact with the body, causing a risk of making the wearability more likely to deteriorate. On the other hand, if the lateral length of the attachment means is long in a portion that does not overlap the absorbent core in the thickness direction, the portion where the elastic member is fixed is long and this makes the waist member itself stiff, causing a risk of making the texture more likely to deteriorate.

The present invention was achieved in light of conventional problems such as that described above, an aspect of the present invention is to provide an underpants-shaped absorbent article which has good texture while improving the wearability of an absorbent core.

### [SOLUTION TO PROBLEM]

A main aspect of the present invention of achieving the above-described aspect is an underpants-shaped absorbent article having a vertical direction, a lateral direction, and a front-back direction that intersect each other, the underpants-shaped absorbent article including: an absorbent main body that has a liquid-absorbent absorbent core; a waist member; and a first elastic member and a second elastic member on at least a one side of the waist member in the front-back direction, the first elastic member being arranged at a position that does not overlap the absorbent core with respect to the vertical direction and being capable of stretching/contracting in the lateral direction, the second elastic member being arranged at a position that overlaps the absorbent core with respect to the vertical direction and being capable of stretching/contracting in the lateral direction, in a region where the waist member in the stretched state and the absorbent core overlap with respect to the lateral direction, a total lateral length of an attachment means for attaching the first elastic member to the waist member being shorter than a total lateral length of an attachment means for attaching the second elastic member to the waist member. Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article which has good texture while improving the wearability of an absorbent core.

### BRIEF DESCRIPTION OF DRAWINGS

FIG. 1 is a schematic perspective view showing an example of the configuration of a diaper 1.
FIG. 2 is a schematic plan view of the diaper 1 in a stretched state as viewed from a skin side in a thickness direction.
FIG. 3 is a schematic cross-sectional view taken along line A-A of FIG. 2.
FIG. 4 is a plan view and a cross-sectional view of an absorbent main body 10.
FIG. 5 is a diagram illustrating the range in a waist member 20 within which a plurality of welded portions 50 are formed.
FIG. 6 is a diagram illustrating an example of the arrangement of the welded portions 50.
FIGS. 7A and 7B are diagrams illustrating the principle by which waist elastic members 25 are attached to the waist member 20 by the welded portions 50.
FIG. 8 is a diagram illustrating the range in the waist member 20 within which adhesive portions 70 for attaching leg elastic members 27 are formed.
FIG. 9 is a plan view illustrating the back side of the diaper 1 in the stretched state.
FIG. 10 is a schematic plan view of a diaper 2 according to a modified example in the unfolded and stretched state.

### DESCRIPTION OF EMBODIMENTS

At least the following matters will be clear with the description of this specification and the attached drawings.

### (Aspect 1)

An underpants-shaped absorbent article having a vertical direction, a lateral direction, and a front-back direction that intersect each other, the underpants-shaped absorbent article including: an absorbent main body that has a liquid-absorbent absorbent core; a waist member; and a first elastic member and a second elastic member on at least a one side of the waist member in the front-back direction, the first elastic member being arranged at a position that does not overlap the absorbent core with respect to the vertical direction and being capable of stretching/contracting in the lateral direction, the second elastic member being arranged at a position that overlaps the absorbent core with respect to the vertical direction and being capable of stretching/contracting in the lateral direction, in a region where the waist member in the stretched state and the absorbent core overlap with respect to the lateral direction, a total lateral length of an attachment means for attaching the first elastic member to the waist member being shorter than a total lateral length of an attachment means for attaching the second elastic member to the waist member.

According to the underpants-shaped absorbent article of aspect 1, the length of the attachment means of the second elastic member is relatively longer, and the second elastic member becomes less likely to stretch excessively. Therefore, at the time of putting on of the absorbent article, when pulling up the waist member upward, a force is more likely to act on the absorbent core. This makes the absorbent core likely to fit to the wearer's crotch region. Further, the length of the attachment means of first elastic member is relatively short, and therefore it prevents the stiffness of the waist member from becoming excessively high. This increases the wearability of the absorbent core, enabling to making good texture likely to be realized.

### (Aspect 2)

The underpants-shaped absorbent article according to aspect 1, wherein a part of the second elastic member is arranged extending in a direction that intersects the lateral direction.

According to the underpants-shaped absorbent article of aspect 2, the stress exerted by the second elastic member includes a component along the vertical direction. Therefore, when the waist member is pulled upward at the time of putting on, the absorbent core is more likely to be pulled upward by the contractive force of the second elastic member. This makes the absorbent core likely to be pulled up, making it possible to improve the wearability.

### (Aspect 3)

The underpants-shaped absorbent article according to aspect 1 or 2, wherein the second elastic member is attached to the waist member by the attachment means in which an adhesive is used.

According to the underpants-shaped absorbent article of aspect 3, the integrity between the waist member and the second elastic member increases. Therefore, the second elastic member and the waist member work together when putting on the absorbent article, and this can make it likely to pull up the absorbent core. In addition, by the attachment means in which the adhesive is used, it is possible to fix the second elastic member along the desired path. Accordingly, it increases the freedom of design and can make it likely to freely give the stretchability to the waist member, even in the case where it includes a section that intersects the lateral direction.

### (Aspect 4)

The underpants-shaped absorbent article according to any one of aspects 1 to 3, wherein the second elastic member is attached to the waist member in a region outside the absorbent core in the lateral direction by the attachment means in which the adhesive is used.

According to the underpants-shaped absorbent article of aspect 4, the integrity between the second elastic member and the waist member increases outside the absorbent core in the lateral direction. Therefore, the force for pulling the waist member in the lateral direction and lifting it up becomes more likely to act on to the absorbent core. This makes the force more likely to act on the absorbent core efficiently through the second elastic member, and can make it easier to pull up the absorbent core.

### (Aspect 5)

The underpants-shaped absorbent article according to any one of aspects 1 to 4, wherein the underpants-shaped absorbent article has a pair of leg openings, and the second elastic member is attached to the waist member in a region that extends along a contour of each of the leg openings, by the attachment means in which the adhesive is used.

According to the underpants-shaped absorbent article of aspect 5, the second elastic member is formed integrally with the waist member along the contour of the leg openings. Therefore, when putting on absorbent article, the force for pulling up the waist member becomes more likely to act on the leg openings as well, making it possible to prevent the leg openings from getting caught on the wearer's legs. This make it more likely to pull up the waist member and the absorbent core.

### (Aspect 6)

The underpants-shaped absorbent article according to any one of aspects 1 to 5, wherein the waist member has a first sheet and a second sheet that is overlaid on a non-skin side with respect to the first sheet, the first sheet and the second sheet are joined to each other by a plurality of welded portions that are dispersed, and the first elastic member is attached to the waist member by being sandwiched between two of the welded portions that are adjacent in the vertical direction.

According to the underpants-shaped absorbent article of aspect 6, the welded portions that join the first sheet and the second sheet are formed in a dispersed manner, and the welded portions can cause the first elastic member to be attached. This makes it likely to maintain the flexibility and the stretchability of the waist member. In addition, the first sheet and the second sheet are joined by the welded portions, and the joining strength of the waist member is more likely to be secured. This makes the waist member less likely to tear and makes the sheets less likely to peel off from each other.

### (Aspect 7)

The underpants-shaped absorbent article according to aspect 6, wherein the waist member has a plurality of the first elastic members spaced apart in the vertical direction, and the waist member has the welded portion between two of the first elastic members that are adjacent in the vertical direction, the welded portion not being contact with the two of the first elastic members.

According to the underpants-shaped absorbent article of aspect 7, the welded portion is provided in portions that do not contribute to the attaching of the first elastic member, thereby further enhancing the joining strength of the first sheet and the second sheet that constitute the waist member. Therefore, even if the waist member is pulled when putting on the absorbent article, it can make it likely to further prevent the waist member from being torn, and the like.

### (Aspect 8)

The underpants-shaped absorbent article according to aspect 6 or 7, wherein the second elastic member is sandwiched between the first sheet and a third sheet that is overlaid on a non-skin side with respect to the first sheet, and the second sheet and the third sheet have a portion where the second sheet and the third sheet overlap with respect to the vertical direction.

According to the underpants-shaped absorbent article in aspect 8, in the portion where the second sheet and the third sheet overlap, at least three layers of the sheet member (first sheet to third sheet) are overlaid in the thickness direction, and this increases the strength compared to the rest of the waist member. This can make it easier to prevent the waist member from tearing when the waist member is pulled up at the time of putting on the absorbent article.

### (Aspect 9)

The underpants-shaped absorbent article according to any one of aspects 6 to 8, wherein a portion where the first sheet and the second sheet are joined and a portion where the second sheet and the third sheet are joined partially overlap with respect to the vertical direction.

According to the underpants-shaped absorbent article of aspect 9, this makes the first to third sheets likely to integrate, making it possible to increase the strength of the overlapping portion. This can make the waist member further less likely to tear.

### (Aspect 10)

The underpants-shaped absorbent article according to any one of aspects 6 to 9, wherein the underpants-shaped absorbent article has a welded portion row in which a plurality of the welded portions are located spaced apart in the vertical direction, and at least a part of the welded portion row is arranged straddling an outer end of the absorbent main body in the lateral direction.

According to the underpants-shaped absorbent article of aspect 10, the strength of the boundary portion between the waist member and the absorbent main body (the portion that overlap the two side ends of the absorbent main body) is increased. Therefore, the strength of the waist member is reinforced at the boundary portion where stress tends to concentrate, making it possible to improve the durability of the waist member.

### (Aspect 11)

The underpants-shaped absorbent article according to any one of aspect 6 to 10, wherein the underpants-shaped absorbent article has a fold-back portion in an upper end portion of the waist member, the fold-back portion being a portion in which the first sheet and second sheet that are overlaid are folded together from above to below in the vertical direction.

According to the underpants-shaped absorbent article in aspect 11, in the upper end portion, there is a portion in which four layers of sheet members are overlaid when the waist member is viewed in the thickness direction. As a result, the strength of the waist member at the upper end portion increases locally, and this makes it likely to prevent the waist member from tearing or stretching excessively when holding and pulling up the upper end portion.

### (Aspect 12)

The underpants-shaped absorbent article according to aspect 11, wherein, in the fold-back portion, at least parts of the first sheet and the second sheet are joined to each other by the welded portion.

According to the underpants-shaped absorbent article in aspect 12, in the fold-back portion, the first sheet and the second sheet are more likely to be integrated, resulting in greater strength. This prevents the two sheets of the sheet members from peeling off or one sheet of them from tearing when pulling the waist member, and this makes it possible to improve the wearability.

### (Aspect 13)

The underpants-shaped absorbent article according to any one of aspects 6 to 12, wherein the underpants-shaped absorbent article has a tape member that is arranged in a non-skin-side surface of the waist member, and at least a part of the welded portion that joins the first sheet and the second sheet has a portion that overlaps the tape member with respect to the vertical direction and the lateral direction.

According to the underpants-shaped absorbent article in aspect 13, the stiffness of the waist member is higher in the portion where the tape member and the welded portion overlap when the waist member is viewed in the thickness direction compared to the non-overlapping portion. This prevents the waist member from being excessively stretched or torn when pulling the waist member at the time of putting on the absorbent article, and this can improve the wearability.

### (Aspect 14)

An underpants-shaped absorbent article having a vertical direction, a lateral direction, and a front-back direction that intersect each other, the underpants-shaped absorbent article including: an absorbent main body that has a liquid-absorbent absorbent core; a waist member; and a first elastic member and a second elastic member on at least a one side of the waist member in the front-back direction, the first elastic member being arranged at a position that does not overlap the absorbent core with respect to the vertical direction and being capable of stretching/contracting in the lateral direction, the second elastic member being arranged below the first elastic member in the vertical direction and being capable of stretching/contracting in the lateral direction, the second elastic member having a curved portion that is curved inward from outside in the lateral direction and from above to below in the vertical direction, in a region between a lateral one end and a lateral other end of the waist member in the lateral direction, a total lateral length of an attachment means for attaching the first elastic member to the waist member being shorter than a total lateral length of an attachment means for attaching the second elastic member to the waist member.

According to the underpants-shaped absorbent article of aspect 14, the length of the attachment means of the second elastic member is relatively longer, and the second elastic member becomes less likely to stretch excessively. Therefore, at the time of putting on of the absorbent article, when pulling up the waist member upward, a force is more likely to act on the absorbent core. This makes the absorbent core likely to fit to the wearer's crotch region. Further, the length of the attachment means of first elastic member is relatively short, and therefore it prevents the stiffness of the waist member from becoming excessively high. This increases the wearability of the absorbent core, enabling to making good texture likely to be realized.

### (Aspect 15)

The underpants-shaped absorbent article according to aspect 14, wherein the second elastic member has a low stretchy region in at least a part of a region that overlaps the absorbent core with respect to the lateral direction, the low stretchy region having a stretchability that is lower than a remaining region, and, in a region outside the low stretchy region in the lateral direction, a total length of the attachment means for attaching the first elastic member to the waist member is shorter than a total length of the attachment means for attaching the second elastic member to the waist member.

According to the underpants-shaped absorbent article of aspect 15, the curved portion is more likely to be firmly fixed to the waist member on the two lateral sides of the absorbent core, making a force more likely to transmit in the vertical direction (in the direction intersecting the lateral direction). Therefore, when pulling the waist member upward, the force for pulling the waist member is more likely to act on the absorbent core, making it possible to improve the wearability of the absorbent core 11.

### (Aspect 16)

The underpants-shaped absorbent article according to aspect 14 or 15, wherein the second elastic member is attached to the waist member by the attachment means in which an adhesive is used.

According to the underpants-shaped absorbent article of aspect 16, the integrity between the waist member and the second elastic member increases. Therefore, the second elastic member and the waist member work together when putting on the absorbent article, and this can make it likely to pull up the absorbent core. In addition, by the attachment means in which the adhesive is used, it is possible to fix the second elastic member along the desired path. Accordingly, it increases the freedom of design and can make it likely to freely give the stretchability to the waist member, even in the case where it includes a curved portion that intersects the lateral direction.

### (Aspect 17)

The underpants-shaped absorbent article according to any one of aspects 14 to 16, wherein the waist member has a first sheet and a second sheet that is overlaid on a non-skin side with respect to the first sheet, the first sheet and the second sheet are joined to each other by a plurality of welded portions that are dispersed, and the first elastic member is attached to the waist member by being sandwiched between two of the welded portions that are adjacent in the vertical direction.

According to the underpants-shaped absorbent article of aspect 17, the welded portions that join the first sheet and the second sheet are formed in a dispersed manner, and the welded portions can cause the first elastic member to be attached. This makes it likely to maintain the flexibility and the stretchability of the waist member. In addition, the first sheet and the second sheet are joined by the welded portions, and the joining strength of the waist member is more likely to be secured. This makes the waist member less likely to tear and makes the sheets less likely to peel off from each other.

### Embodiment

As an example of the underpants-shaped absorbent article according to the embodiment of the present invention, an underpants-shaped diaper 1 (hereinafter, simply referred to as "diaper 1") will be described. In addition, the underpants-shaped absorbent article also includes a shorts-type sanitary napkin and an underpants-shaped absorbent pad.

### Basic structure of diaper 1

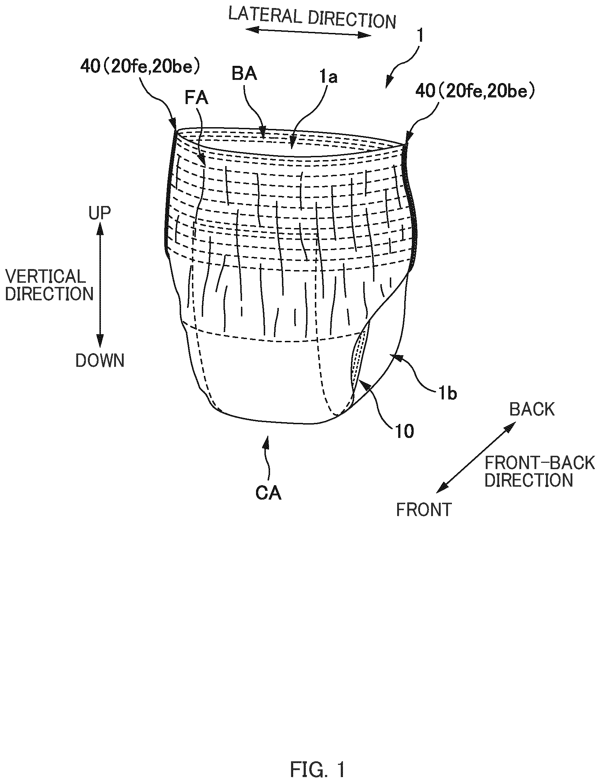

FIG. 1 is a schematic perspective view showing an example of the configuration of the diaper 1 according to the present embodiment.

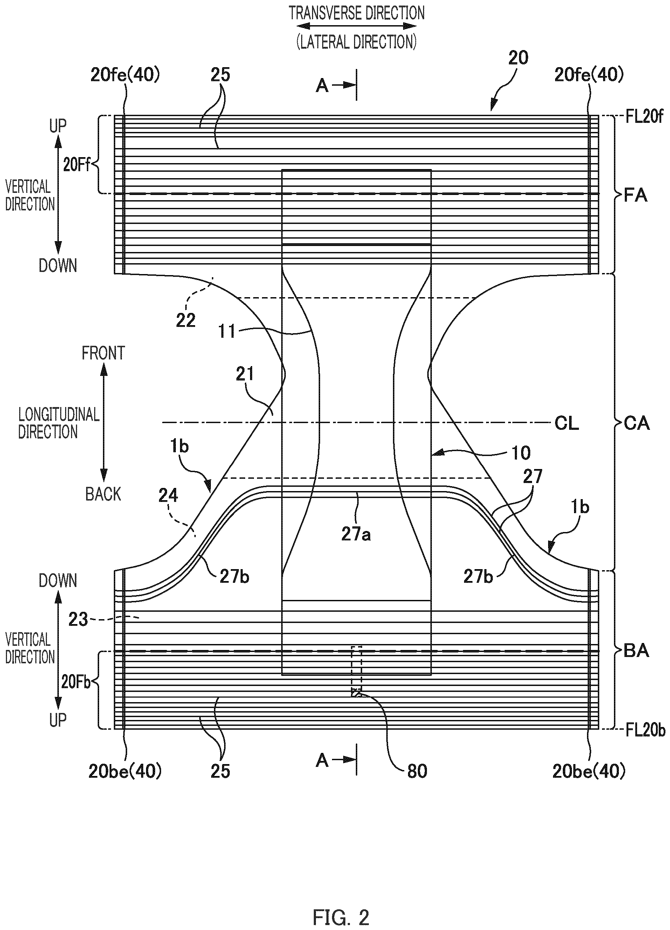

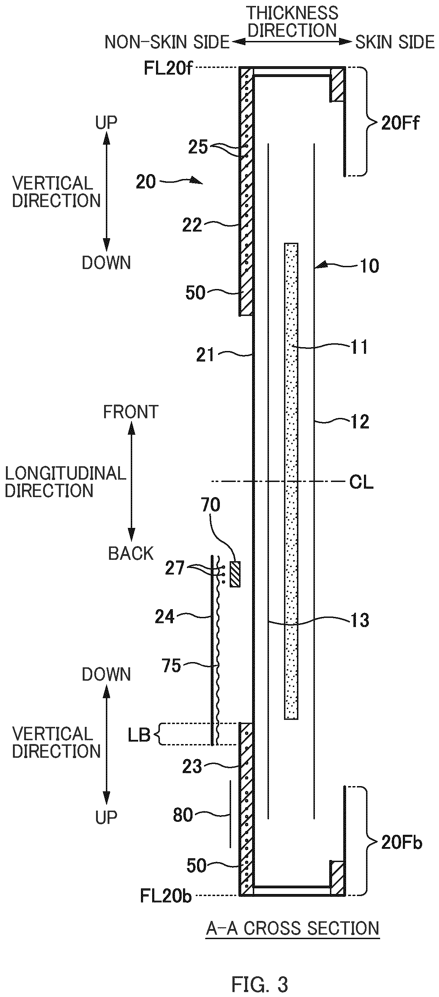

FIG. 2 is a schematic plan view of the diaper 1 in a stretched state as viewed from a skin side in a thickness direction. FIG. 3 is a schematic cross-sectional view taken along line A-A of FIG. 2.

In addition, the "stretched state" of the diaper 1 refers to a state where the entire diaper 1 (entire product) is stretched without wrinkles by stretching the elastic members (e.g., later-described waist elastic members 25 and leg elastic members 27) provided in the diaper 1, or more specifically, a state where the diaper 1 is stretched until the dimensions of the constituent members of the diaper 1 (e.g., a later-described absorbent main body 10 and waist member 20) match or are close to the dimensions of the members on their own. On the other hand, compared to the stretched state, the natural state refers to the state shown in FIG. 1. The "natural state" is a state where the diaper 1 has been left alone for a predetermined period. For example, the waist member 20 of the diaper 1 is pulled outward on both sides in the lateral direction to put the waist member 20 in the "stretched state", and the stretched state is maintained for 15 seconds, and then the diaper 1 is released and placed on a flat surface such as a desk.

Then, the state after five minutes of being laid flat on the flat surface is defined as the natural state.

In the underpants-shaped state shown in FIG. 1, the diaper 1 has a "vertical direction", a "lateral direction", and a "front-back direction" that are orthogonal to each other. Also, as shown in FIG. 3, the diaper 1 has a "thickness direction", which is the direction in which members are overlaid on each other. When the wearer wears the diaper 1, the "upper side" in the vertical direction is the side that faces the wearer's torso, and the "lower side" in the vertical direction is the side that faces the wearer's crotch region. Also, in the put-on state, the "front side" in the front-back direction is the side that faces the wearer's abdomen, and the "back side" in the front-back direction is the side that faces the wearer's back. In addition, when the wearer wears the diaper 1, the "skin side" in the thickness direction is the side that comes into contact with the wearer's skin, and the "non-skin side" in the thickness direction is the opposite side.

In addition, in the unfolded state shown in FIG. 2, the diaper 1 has a "longitudinal direction" and a "transverse direction" that are orthogonal to each other. The "longitudinal direction" is a direction conforming to the vertical direction in the underpants-shaped state, and the "transverse direction" is a direction conforming to the lateral direction in the underpants-shaped state.

The diaper 1 of the present embodiment includes: an absorbent main body 10 that has liquid absorbency and absorbs a liquid such as urine; and the waist member 20 that is joined to the non-skin side of the absorbent main body 10 and is arranged at the wearer's waist when the diaper 1 is put on.

### Absorbent main body 10

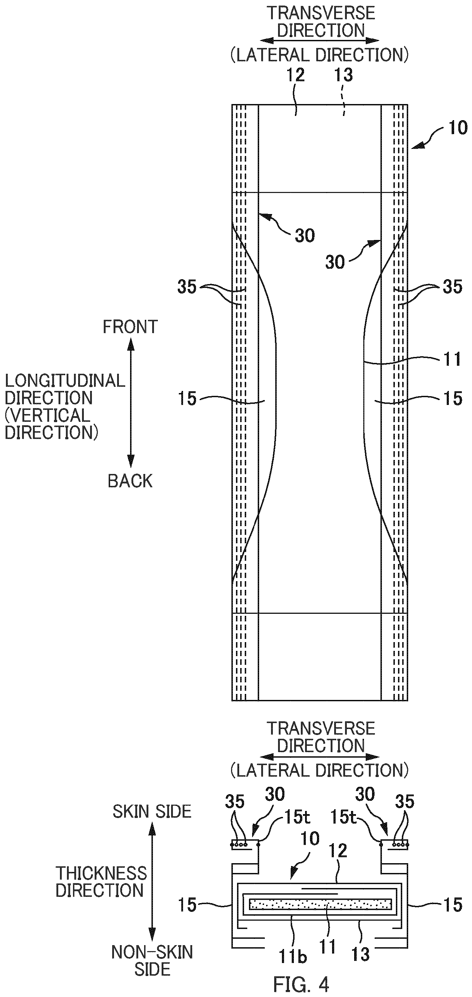

FIG. 4 is a plan view and a cross-sectional view of an absorbent main body 10. As shown in FIG. 2, the absorbent main body 10 of the present embodiment has a substantially rectangular shape in a plan view, and the longitudinal direction (i.e., the long side direction of the absorbent main body 10) conforms to the vertical direction of the diaper 1. As shown in FIGS. 3 and 4, the absorbent main body 10 includes an absorbent core 11 that extends in the longitudinal direction (vertical direction), a top sheet 12 arranged on the skin side with respect to the absorbent core 11, a back sheet 13 arranged on the non-skin side with respect to the absorbent core 11, and a pair of side sheets 15 and 15 arranged on both sides of the absorbent core 11 in the transverse direction (lateral direction).

The absorbent core 11 is a member that absorbs and retains excreted fluid such as urine. For example, the absorbent core 11 can be obtained by molding liquid-absorbent fibers (e.g., pulp) mixed with a superabsorbent polymer (SAP) into a predetermined shape. The absorbent core 11 of the present embodiment is shaped so as to have a substantially hourglass shape with its longitudinal central portion narrowing inward in the lateral direction, as shown in FIG. 4, but the shape of the absorbent core 11 is not limited to this. In addition, the outer circumferential surface of the absorbent core 11 may be covered by a liquid-permeable sheet member (core-wrapping sheet 11b) made of tissue paper or the like.

The top sheet 12 is a liquid-permeable sheet member that can come into contact with the wearer's skin during usage, and is made of hydrophilic air-through nonwoven fabric or the spunbond nonwoven fabric, for example. In the cross-sectional view of FIG. 4, the top sheet 12 is arranged so that its two lateral end portions are folded onto the non-skin side with respect to the absorbent core 11, and therefore the top sheet 12 wraps around and covers the absorbent core 11 from the skin side. In addition, another sheet or the like (not shown) may be provided between the top sheet 12 and the absorbent core 11.

The back sheet 13 is a liquid-impermeable and breathable sheet member (breathable sheet member) for preventing a liquid such as urine absorbed in the absorbent core 11 from leaking to the outside, and is made of a liquid-impermeable film such as polyethylene, polypropylene or the like.

Each of the side sheets 15 is a sheet member located on both transverse sides of the absorbent core 11, and is made of a hydrophobic nonwoven fabric sheet such as a SMS (spunbondmeltblown-spunbond) nonwoven fabric sheet, for example. The vertical (lateral) one-end side of the side sheet 15 is folded so as to be located on the non-skin side with respect to the absorbent core 11 and the back sheet 13. On the other hand, the vertical (lateral) other-end side of the side sheet 15 is folded so as to be located on the skin side with respect to the absorbent core 11 and the top sheet 12, forming a pair of leak-proof wall portions 30 and 30 on both sides of the absorbent main body 10.

The pair of leak-proof wall portions 30 are respectively provided on the two sides of the absorbent main body 10 in the transverse direction (lateral direction), and are portions corresponding to so-called barrier cuffs. When the diaper 1 is put on, the leak-proof wall portions 30 rise toward the wearer's skin on the two sides of the absorbent main body 10 in the lateral direction, thus suppressing the case where excreted fluid such as urine leaks along the wearer's skin to the outside of the absorbent main body 10 in the lateral direction. In addition, each of the leak-proof wall portions 30 may be composed of two or more sheet members (side sheet 15).

As shown in the cross-sectional view of FIG. 4, in the end portion 15t of the portion of the side sheet 15 that is folded toward the skin side in the thickness direction, the side sheet 15 is folded over multiple times so as to be overlaid in the thickness direction, and the leak-proof-wall elastic members 35 are provided between the overlaid portions of the side sheet 15 in a state of being stretched along the longitudinal direction. When the diaper 1 is put on, the end portions 15t of the leak-proof wall portions 30 contracts in the longitudinal direction (vertical direction) due to the stretchability exhibited by the leak-proof-wall elastic members 35, making the absorbent main body 10 likely to fit around the wearer's legs.

### Waist member 20

The waist member 20 is a sheet member located on the non-skin side of the absorbent main body 10, and has a skin-side sheet 21, non-skin-side sheets 22 and 23, and a cover sheet 24. Also, as shown in FIG. 2, the portion of the waist member 20 that overlaps the later-described side joining portion 40 provided on the front side in the longitudinal direction with respect to the center position CL (on the front side in the front-back direction in FIG. 1) is referred to as a "front waist portion FA". Similarly, the portion of the waist member 20 that overlaps the side joining portion 40 provided on the back side in the longitudinal direction with respect to the center position CL (on the back side in the front-back direction in FIG. 1) is referred to as a "back waist portion BA". In the longitudinal direction, the portion between the back end of the front waist portion FA and the front end of the back waist portion BA is referred to as a "crotch portion CA".

The skin-side sheet 21 is a sheet member that is arranged on the skin side of the waist member 20, and is made of spunbond nonwoven fabric, SMS nonwoven fabric, or the like, for example. As shown in FIG. 2, the skin-side sheet 21 extends continuously from the front side to the back side in the longitudinal direction, and has a shape narrowed inward in the lateral direction in the crotch portion CA. This narrowed portion becomes the leg openings 1b (described later) in the diaper 1 in the underpants-shaped state.

The non-skin-side sheet 22 is a sheet member overlaid on the non-skin side of the skin-side sheet 21, on the front side in the longitudinal direction. Similarly to the skin-side sheet 21, the non-skin-side sheet 22 is made of spunbond nonwoven fabric, SMS nonwoven fabric, or the like. The skin-side sheet 21 and the non-skin-side sheet 22 are overlaid in the thickness direction and joined to each other by a plurality of welded portions 50, 50, ..., which will be described later. In addition, between the skin-side sheet 21 and the non-skin-side sheet 22, a plurality of the waist elastic members 25, 25, ... are provided. The plurality of waist elastic members 25, 25,... are arranged side-by-side spaced apart in the longitudinal direction (vertical direction), and are attached between the skin-side sheet 21 and the non-skin-side sheet 22 in a state of being stretched in the transverse direction (lateral direction). As a result, the waist elastic members 25 exhibit the stretchability along the transverse direction (lateral direction), which then gives the stretchability to the front waist portion FA of the waist member 20.

The waist elastic members 25 can be made of, for example, an elastic resin material whose main component is thermoplastic elastic resin, which is molded into the shape of an elastic string. As the above-mentioned thermoplastic elastic resin, a thermoplastic resin that exhibits rubber elasticity at room temperature is preferable, for example, and an example thereof is thermoplastic elastomers described in JIS K 6418:2007. As the above-mentioned thermoplastic elastic resin, a material with a temperature range in which elastic deformation occurs of approximately 100°C or less is preferable. Examples of the above-mentioned thermoplastic elastic resin include olefin elastomers (e.g., VERSIFY (trademark) from The Dow Chemical Company), propylene elastomers (e.g., Vistamaxx (trademark) from Exxon Mobil Corporation), and styrene elastomers (e.g., Quintac (trademark) from Zeon Corporation).

The non-skin-side sheet 23 is a sheet member overlaid on the non-skin side of the skin-side sheet 21, on the back side in the longitudinal direction, and has a similar configuration to the non-skin-side sheet 22. That is, the non-skin-side sheet 23 is joined to the skin-side sheet 21 by the plurality of welded portions 50. In addition, between the skin-side sheet 21 and the non-skin-side sheet 23, a plurality of the waist elastic members 25, 25, ... are provided in a manner of being capable of stretching/contracting along the transverse direction (lateral direction).

The cover sheet 24 is a sheet member overlaid on the non-skin side of the skin-side sheet 21, similarly to the non-skin-side sheet 23, on the back side in the longitudinal direction. As shown in FIG. 3, in the diaper 1, the cover sheet 24 is located below the non-skin-side sheet 23 in the vertical direction (on the front side in the longitudinal direction), and a part thereof is arranged overlapping the non-skin-side sheet 23. Hereinafter, the portion where the cover sheet 24 and the non-skin-side sheet 23 overlap with respect to the vertical direction (longitudinal direction) is also referred to as a "back overlapping portion LB".

The cover sheet 24 is joined to a part of the skin-side sheet 21 and a part of the non-skin-side sheet 23 via the adhesive surface 75, which is made of an adhesive such as a hot-melt adhesive or the like. Between the cover sheet 24 and the skin-side sheet 21 in the thickness direction, there are provided the leg elastic members 27, which are made of elastic strings or the like (see FIG. 3). The leg elastic members 27 each have a straight portion 27a in the transverse (lateral) center and curved portions 27b on the two sides in the transverse direction. In the straight portion 27a, the portion where the leg elastic member 27 overlaps the absorbent main body 10 (absorbent core 11) is arranged in a straight line along the transverse direction. In the curved portion 27b, the leg elastic member 27 is arranged in a curved manner along the contour of the leg opening 1b. The stretchability exhibited by the leg elastic members 27 makes the leg openings 1b likely to fit around the wearer's legs on the back side when the diaper 1 is put on, and also makes the portion of the waist member 20 covering the wearer's buttocks less likely to roll up.

In addition, in the upper end portion of the waist member 20 in the vertical direction, a part of the skin-side sheet 21 and parts of the non-skin-side sheet 22 and 23 are folded downward. In FIG. 3, the skin-side sheet 21 and the non-skin-side sheet 22 form a front fold-back portion 20Ff, which is a portion folded back downward in the vertical direction and toward the skin side in the thickness direction, at the front bending position FL20f located in the front upper end portion of the waist member 20. The front fold-back portion 20Ff is joined to at least portions of sheet members located adjacent in the thickness direction (the skin-side sheet 21 and the absorbent main body 10, the top sheet 12 in FIG. 3) with an adhesive such as a hot-melt adhesive.

Similarly, in FIG. 3, the skin-side sheet 21 and the non-skin-side sheet 23 form a back fold-back portion 20Fb, which is a portion folded back downward in the vertical direction (toward the front side in the longitudinal direction) and toward the skin side in the thickness direction, at the back bending position FL20b located in the back upper end portion of the waist member 20. The back fold-back portion 20Fb is joined to at least portions of sheet members adjacent in the thickness direction (the skin-side sheet 21 and the absorbent main body 10, the top sheet 12 in FIG. 3) with an adhesive such as a hot-melt adhesive.

In the diaper 1, the front fold-back portion 20Ff may be formed by folding back only either sheet member of the skin-side sheet 21 or the non-skin-side sheet 22, at the front bending position FL20f. Similarly, the back fold-back portion 20Fb may be formed by folding back only either sheet member of the skin-side sheet 21 or the non-skin-side sheet 23, at the back bending position FL20b. That is, the fold-back portions 20Ff and 20Fb each may be formed by one sheet member.

Also, a separate sheet member may be provided on the skin side with respect to the fold-back portions 20Ff and 20Fb. For example, by arranging this sheet member so that it straddles the vertical lower ends of the fold-back portions 20Ff and 20Fb in the vertical direction from the skin side (not shown in FIG. 3), it is possible to prevent the lower end edges of the fold-back portions 20Ff and 20Fb from digging into the wearer's skin when the diaper 1 is put on.

The diaper 1 is folded one time from the unfolded state shown in FIG. 2, and at this stage, the absorbent main body 10 and the waist member 20 is folded in the longitudinal direction at a folding position, which is the longitudinal center position CL (shown as the single-dotted chain line in FIG. 2). In this folded state, of the waist member 20, the front waist portion FA and the back waist portion BA are overlaid on each other in the front-back direction. The transverse side portions 20fe and 20fe of the front waist portion FA and the transverse side portions 20be and 20be of the back waist portion BA are joined together using a known joining means such as seal welding, thus forming a pair of the side joining portions 40 and 40. As a result, the two portions of the folded waist member 20 are connected on the front side and the back side to form a ring, thus forming one waist opening 1a and the pair of leg openings 1b as shown in FIG. 1, and obtaining the underpants-shaped diaper 1.

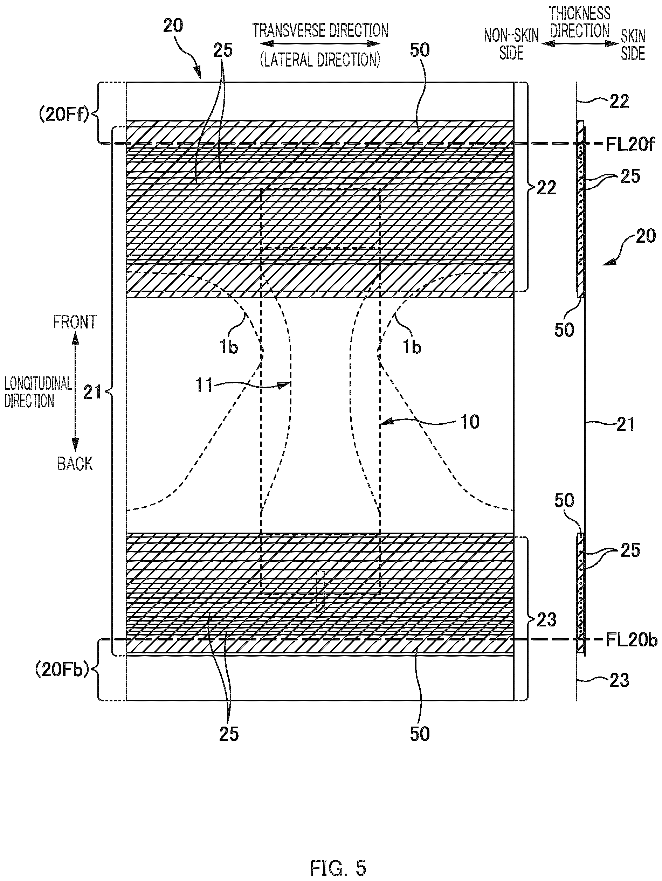

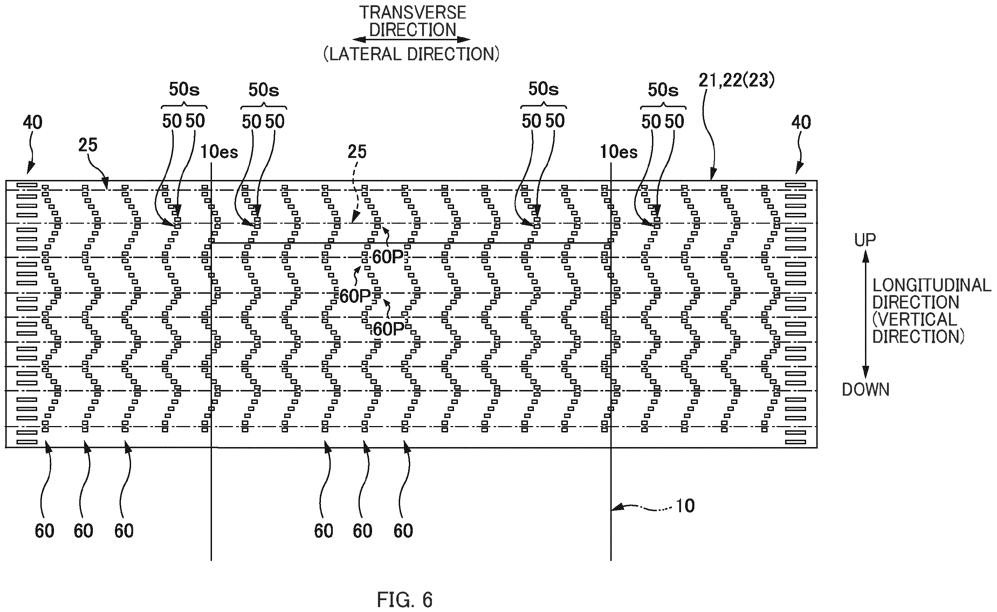

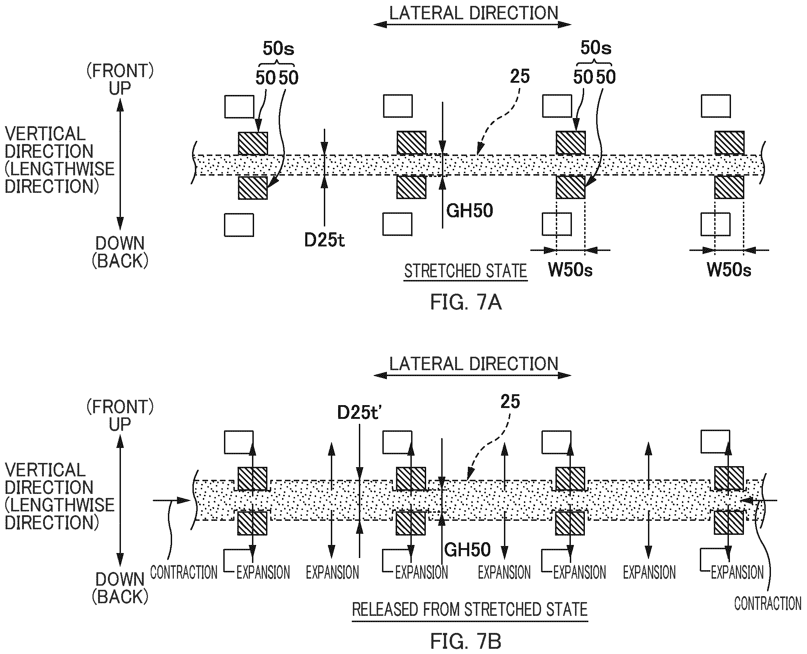

Attaching of waist elastic members 25 and leg elastic members 27 The specific method for attaching the waist elastic members 25 and the leg elastic members 27 to the waist member 20 will be explained. First, the method for attaching the waist elastic members 25 will be described. FIG. 5 is a diagram illustrating the range in the waist member 20 within which a plurality of the welded portions 50 are formed. FIG. 6 is a diagram illustrating an example of the arrangement of the welded portions 50. FIGS. 7A and 7B are diagrams illustrating the principle by which the waist elastic members 25 are attached to the waist member 20 by the welded portions 50.

In the waist member 20 of the diaper 1, the skin-side sheet 21 and the non-skin-side sheets 22 and 23 are joined by the plurality of welded portions 50, 50, ... that are formed by using a known welding means such as ultrasonic welding. In FIG. 5, the hatched portion is the region where the welded portions 50 are formed when the non-skin-side sheets 22 and 23 (corresponding to the second sheet) are joined to the non-skin side of the skin-side sheet 21 (corresponding to the first sheet) during the manufacturing process of diaper 1. At the stage of joining the skin-side sheet 21 and the non-skin-side sheets 22 and 23, the leg openings 1b have not yet been formed (the sheet member has not yet been cut). In addition, in FIG. 5, the position of the absorbent main body 10 (absorbent core 11) is indicated by a dotted line, but at the stage of forming the welded portions 50, the absorbent main body 10 has not yet been joined to the waist member 20.

As shown in FIG. 5, the skin-side sheet 21 and the non-skin-side sheets 22 and 23 are joined in the portions where they overlap in the thickness direction in the stretched state before being folded at the front bending position FL 20f and the back bending position FL 20b. That is, the welded portions 50 are formed in ranges respectively straddling the folding positions FL20f and FL20b in the front-back direction. In the range indicated by the hatched portions in FIG. 5, between the skin-side sheet 21 (first sheet) and the non-skin-side sheets 22 and 23 (second sheet) that face each other in the thickness direction, the waist elastic members 25, 25, ... which can stretch/contract along the lateral direction are attached due to the welded portions 50, side-by-side spaced apart in the vertical direction (longitudinal direction).

In the waist member 20 of the present embodiment, as shown in FIG. 6, the plurality of welded portions 50, 50, ... are arranged side-by-side spaced apart in the vertical direction (longitudinal direction), thereby forming a welded portion row 60 extending along the vertical direction. A plurality of the welded portion rows 60 are provided spaced apart in the lateral direction (transverse direction). In FIG. 6, for the sake of simplicity, the arrangement and quantities of the waist elastic members 25 and the welded portions 50 are shown schematically. Each welded portion row 60 is arranged so as to have convex portions 60P that are convex on the one side or the other side in the lateral direction. That is, since the lateral positions of the plurality of welded portions 50 included in the welded portion row 60 are different from one another, the welded portion row 60 is formed to have projections and recesses as a whole. In the example of FIG. 6, the welded portion row 60 is formed extending from top to bottom in the vertical direction so as to meander leftward and rightward. However, the arrangement of each of the welded portions 50 is not limited to the example of FIG. 6, and the welded portion row 60 may have convex portions 60P only on the one side in the lateral direction. Also, the welded portion row 60 may be arranged in a straight line along the vertical direction without having any bumps in the lateral direction.

Of the plurality of welded portions 50, 50, ... included in the welded portion row 60, the waist elastic member 25 is sandwiched above and below between the two welded portions 50 and 50 adjacent in the vertical direction, thereby attaching the waist elastic member 25 to the waist member 20. That is, the pair of welded portions 50 and 50 arranged on both vertical sides of the waist elastic member 25 form a welded portion pair 50s, and the waist elastic member 25 is attached by the welded portion pair 50s.

As shown in FIG. 7A, a pair of the welded portions 50 and 50 that form the welded portion pair 50s are arranged with a gap GH50 therebetween in the vertical direction. The size of the gap GH50 is set to be equal to or slightly larger than the outer diameter D25t of the waist elastic member 25 (e.g., an elastic string) in a state of being stretched to a target stretch factor in the lateral direction (GH50 ≥ D25t). That is, the waist elastic member 25 in the stretched state is placed between the welded portion pair 50s in the vertical direction. With such a configuration, in the manufacturing process of the diaper 1, the waist elastic members 25 in the stretched state are placed between the skin-side sheet 21 and the non-skin-side sheet 22 (23), and then when the skin-side sheet 21 and the non-skin-side sheet 22 (23) are joined, it is possible to form the welded portions 50 without overlapping the waist elastic members 25. Here, the "stretch factor" refers to the value R (= L1/L0) that indicates the ratio of the total length L1 of the stretched waist elastic member 25 to the total length L0 in a natural no-load state.

Then, when the waist elastic member 25 is relaxed from the stretched state, as shown in FIG. 7B, the waist elastic member 25 expands in the vertical direction while contracting in the lateral direction, and the outer diameter D25t' after expansion becomes larger than the gap GH50 of the welded portion pair 50s in the vertical direction (D25t' > GH50). Therefore, the expansion of the waist elastic member 25 in the vertical direction is restricted between the welded portion pair 50s, and as a result, the waist elastic member 25 is substantially sandwiched between the welded portions 50 and 50 in the vertical direction. As a result, the welded portion pair 50s restricts the positions of the waist elastic member 25 in the vertical direction and the lateral direction. Accordingly the waist elastic member 25 is attached to the waist member 20 in a manner of being capable of stretching/contracting, and the waist member 20 is given the stretchability in the lateral direction.

In addition, in the diaper 1 in the underpants-shaped state, the waist elastic member 25 is joined by the side joining portions 40 in the two lateral end portions of the waist member 20, and therefore even if the waist member 20 is stretched in the lateral direction when the diaper 1 is put on, the waist elastic member 25 does not come off the waist member 20.

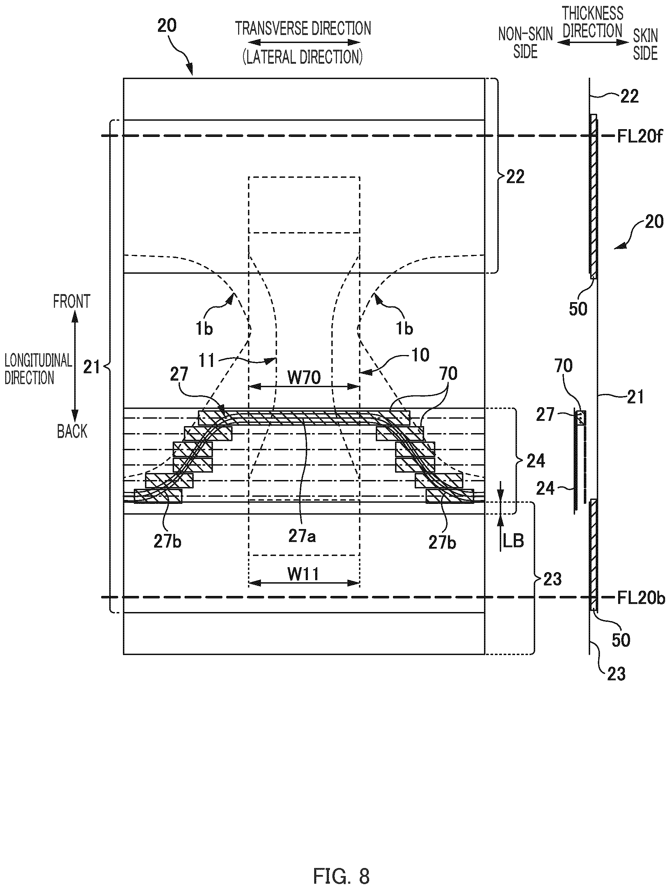

Next, the method for attaching the leg elastic members 27 will be described. FIG. 8 is a diagram illustrating the range in the waist member 20 within which the adhesive portions 70 for attaching the leg elastic members 27 are formed. FIG. 8 shows the state after the skin-side sheet 21 and the non-skin-side sheet 22 (23) have been joined by the welded portions 50 during the manufacturing process of the diaper 1 (see FIG. 5).

The adhesive portions 70 are formed by applying an adhesive such as a hot-melt adhesive, to a predetermined region of the non-skin-side surface of the skin-side sheet 21. In FIG. 8, the adhesive portions 70 are formed by applying the adhesive to a plurality of rectangular regions indicated by hatched portions, along the region where the leg elastic members 27 are to be arranged. The leg elastic members 27 in a state of being stretched in the longitudinal direction (vertical direction) and the transverse direction (lateral direction) as shown in FIG. 8 are placed on the skin-side sheet 21 in which the adhesive portions 70 have been formed, and then the leg elastic members 27 are sandwiched by being covered with the cover sheet 24 (corresponding to the third sheet) from the non-skin side, resulting in being attached to the waist member 20.

In addition, on the skin- side surface of the cover sheet 24, the adhesive surface 75 is formed in advance by applying the adhesive (see FIG. 3), and the opposing surfaces of the skin-side sheet 21 (and part of the non-skin-side sheet 23) and the cover sheet 24 are firmly adhered to each other via the adhesive surface 75.

Also, the following method is acceptable: the adhesive portions 70 is formed on the cover sheet 24 side and the leg elastic members 27 are attached thereto, and subsequently the cover sheet to which the leg elastic members 27 are attached is joined to the non-skin-side surface of the skin-side sheet 21.

By fixing substantially the entirety of the leg elastic members 27 by the adhesive portions 70, it can make the stretchability more likely to exhibit along the contour of the leg openings 1b while maintaining the shapes of the straight portion 27a and the curved portions 27.

### Effects of waist elastic members 25 and leg elastic members 27

In conventional underpants-shaped diapers, each of a plurality of elastic members is attached to the waist member using uniform attachment means. For example, in the case of a conventional underpants-shaped diaper having the waist elastic members 25 and the leg elastic members 27, similar to the diaper 1, it is common that the elastic members 25 and 27 are attached to the waist member by a uniform adhesion pattern using the adhesive, and the like (attachment means). In this case, depending on the length of the portion where the elastic members 25 and 27 are attached, there is a risk of causing a problem of the wearability and the like of the diaper.

For example, in the case of making long the lateral length of a portion where the elastic member is attached and which is located in a region that does not overlap the absorbent core when viewed in the thickness direction, there is a risk that the waist member become stiff in the region where the elastic member is attached, impairing the texture during usage. On the other hand, in the case of making short the lateral length of a portion where the elastic member is attached and which is located in a region that overlaps the absorbent core when viewed in the thickness direction, the elastic member is likely to stretch when pulling up the waist member from the wearer's toe side to the crotch side at the time of putting on the diaper 1, and in some case, this makes the pulling force less likely to act on the absorbent core. In this case, the absorbent core become less likely to be pulled up or to fit to the crotch portion, causing a risk that the wearability deteriorates.

In contrast, in the diaper 1 of the present embodiment, as described in FIGS. 5 to 8, the waist elastic members 25 and the leg elastic members 27 are attached to the waist member 20 using different attachment means (welded portion 50 and adhesive portion 70). This allows the diaper 1 to have good wearability and good texture during usage.

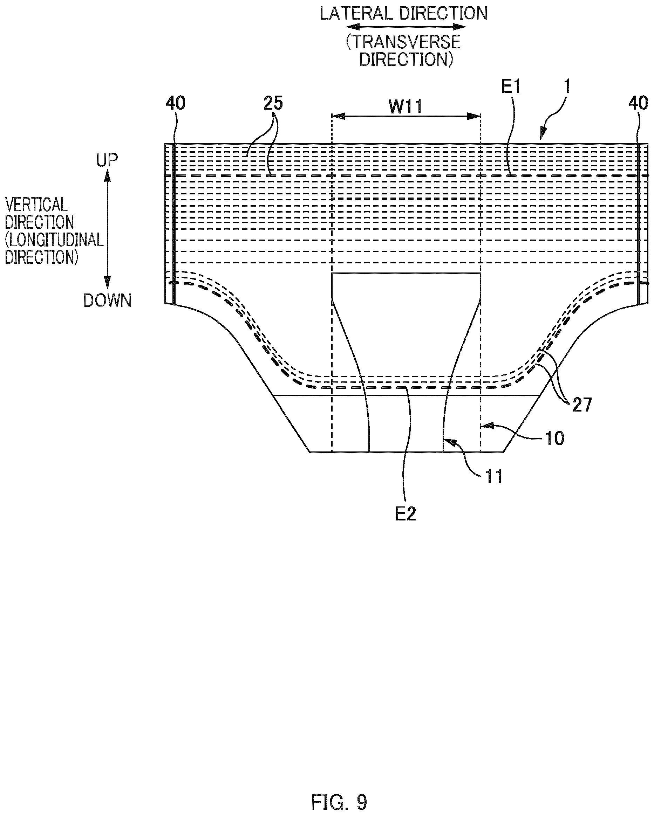

FIG. 9 is a plan view illustrating the back side of the diaper 1 in the stretched state. Among elastic members that are arranged in a region of the diaper 1 that is located on the back side in the front-back direction (on the back side with respect to the longitudinal center position CL in FIG. 2) and that does not overlap the absorbent core 11 with respect to the vertical direction (longitudinal direction), a certain elastic member is defined as a first elastic member E1. In FIG. 9, among the waist elastic members 25 that do not overlap the absorbent core 11, the one indicated by the thick dashed line is the first elastic member E1. Also, among elastic members arranged in a region that overlaps the absorbent core 11 with respect to the vertical direction (longitudinal direction), a certain elastic member is defined as a second elastic member E2. In FIG. 9, among the leg elastic members 27 that overlaps the absorbent core 11, the one indicated by the thick dashed line is the second elastic member E2.

In the diaper 1 of the present embodiment, in the region W11 that overlaps the absorbent core 11 with respect to the lateral direction (transverse direction), the total lateral length of the attachment means for attaching the first elastic member E1 to the waist member 20 (i.e., welded portions 50) is shorter than the total lateral length of the attachment means for attaching the second elastic member E2 to the waist member 20 (i.e., adhesive portions 70) in the region W11 that overlaps the absorbent core 11. The "region that overlaps the absorbent core 11" refers to the range in the lateral direction where the width of the absorbent core 11 is largest (the range indicated by W11 in FIG. 9).

Specifically, in the region W11 that overlaps the absorbent core 11 with respect to the lateral direction, in the case where the first elastic member E1 (waist elastic member 25) is attached by n of the welded portion pairs 50s located side-by-side in the lateral direction and the lateral length of each welded portion pair 50s is defined as a length W50s (see FIG. 7), the total lateral length of the attachment means for attaching the first elastic member E1 is expressed as "n × W50s". In addition, in the region W11 that overlaps the absorbent core 11 with respect to the lateral direction, in the case where the second elastic member E2 (leg elastic member 27) is attached by the adhesive portions 70 extending continuously in the lateral direction and the lateral length of the adhesive portion 70 is a length W70 (see FIG. 8), the total lateral length of the attachment means for attaching the second elastic member E2 is expressed as "W70". And, the total length of the attachment means of the first elastic member E1 (n x W50s) is less than the total length of the attachment means of the second elastic member E2 (W70).

If the total length (n × W50s) of the attachment means of the first elastic member E1 is less than the total length (W70) of the attachment means of the second elastic member E2, the total length of the attachment means (adhesive portion) of the second elastic member E2 is relatively longer. Therefore, compared to the opposite case, the second elastic member E2 is firmly fixed to the waist member 20 and is less likely to stretch excessively. At the time of putting on of the diaper 1, when pulling up the waist member 20 upward, the force for pulling the waist member 20 is more likely to act on the absorbent core 11. In other words, the force for pulling the waist member 20 is more likely to be used to pull up the absorbent core 11 than to stretch the second elastic member E2. This makes the absorbent core 11 likely to fit to the wearer's crotch region. Furthermore, when the diaper 1 is put on, due to the stretchability exhibited by the second elastic member E2, the absorbent core 11, which has a high stiffness, becomes more likely to be pressed against the wearer's skin side. This makes the absorbent core 11 less likely to shift out of position, thereby improving the fit to the body.

In addition, since the total length of the attachment means (welded portions 50) of first elastic member E1 is relatively short, it prevents the stiffness of the waist member 20 from becoming excessively high compared to the opposite case. This increases the flexibility of the waist member 20 and improves the fit, while making the texture softer during usage. In particular, in the region that does not overlap the absorbent core 11, the skin-side surface of the waist member 20 comes into direct contact with the wearer's skin when the diaper 1 is put on, and therefore the flexibility in that region has a significant influence on the texture.

This can make it likely to achieve a good texture when the diaper 1 is put on while improving the wearability of the absorbent core 11. In addition, in FIG. 9, in the case where parts of the waist elastic members 25 are located at positions that overlap the absorbent core 11 with respect to the vertical direction (longitudinal direction), such waist elastic members 25 may be defined as the second elastic member E2.

In addition, in the waist member 20 of the diaper 1, the second elastic member E2 (leg elastic member 27) is partially arranged extending in a direction that intersects the lateral direction. That is, that leg elastic member 27 is arranged along a diagonal direction in the curved portion 27b, and the stress exerted by the leg elastic member 27 includes a component along the vertical direction. Therefore, when the waist member 20 is pulled upward in the vertical direction during putting on of the diaper 1, the absorbent core 11 is more likely to be pulled upward by the contractive force that is exhibited in the curved portion 27b of the leg elastic member 27 and that has a vertical component. This makes the absorbent core 11 likely to be pulled up, making it possible to improve the wearability.

And the second elastic member E2 (leg elastic member 27) is attached to the waist member 20 throughout the lengthwise-direction range by the attachment means (adhesive portion 70) in which an adhesive such as a hot-melt adhesive is used. Thus, similarly to the waist elastic members 25, compared to the case of being attached to the waist member 20 by the dispersed welded portion pairs 50s restricting the movement in the vertical direction and the lateral direction, the integrity of the waist member 20 and the leg elastic member 27 increases. As a result, the second elastic member E2 (leg elastic member 27) and the waist member 20 work together when putting on the diaper 1, and this can make it likely to pull up the absorbent core 11. In addition, by using the adhesive portions 70, it is possible to fix the leg elastic members 27 along the desired path. Accordingly, it increases the freedom of design, and can make it likely to freely give the stretchability to the waist member 20 even in cases where the portion intersecting the lateral direction, such as the curved portion 27b, is included.

In addition, the second elastic member E2 (leg elastic member 27) is attached to the waist member 20 in a region outside the absorbent core 11 in the lateral direction (transverse direction) by the attachment means (adhesive portion 70) in which the above-mentioned adhesive is used. When the waist member 20 is pulled up at the time of putting on the diaper 1, it is necessary to make the legs easier to put through by stretching the waist member 20 toward the two lateral sides. At this time, the integrity between the second elastic member E2 and the waist member 20 increases by the adhesive portions 70, outside the absorbent core 11 in the lateral direction, and therefore a force for pulling the waist member 20 in the lateral direction and lifting it up becomes more likely to act on the absorbent core 11. In the case where the integrity between the second elastic member E2 and the waist member 20 is lower outside the absorbent core 11 in the lateral direction, when pulling the waist member 20 in the lateral direction, the pulling force is more likely to act on the second elastic member E2 to stretch it, causing a risk that the pulling force is less likely to act on the absorbent core 11. In contrast, in the diaper 1 of the present embodiment, the force is more likely to act on the absorbent core 11 efficiently through the second elastic member E2, and can make it easier to pull up the absorbent core 11.

Furthermore, the second elastic member E2 (leg elastic member 27) is attached to the waist member 20, in a region outside the absorbent core 11 in the lateral direction (transverse direction) 11 and extending along the contour of each of the pair of leg openings 1b and 1b, by the attachment means (adhesive portion 70) in which an adhesive is used. When putting on the diaper 1, the wearer's legs are made pass through the leg openings 1b and subsequently the waist member 20 and the absorbent main body 10 are pulled up. At this time, due to frictional force generated between the leg openings 1b and the wearer's legs, there is a case where it is less likely to pull up the waist member 20. In contrast, in the present embodiment, the second elastic member E2 is provided integrally with the waist member 20 along the contour of the leg openings 1b, and this makes the force for pulling up the waist member 20 likely to act on the leg openings 1b as well. This makes it possible to prevent the leg openings 1b from getting caught on the wearer's legs against the frictional force. This make it more likely to pull up the waist member 20 and the absorbent core 11.

In addition, in the front waist portion FA and the back waist portion BA of the waist member 20, the skin-side sheet 21 (first sheet) and the non-skin-side sheet 22, 23 (second sheet) are joined by a plurality of welded portions 50, 50, ... provided dispersed. Between the first sheet and the second sheet in the thickness direction, the waist elastic member 25 (first elastic member E1) is attached by being sandwiched between the pair of welded portion pairs 50s from above and below. In other words, the welded portions 50 that join the first sheet and the second sheet are formed in a dispersed manner and cause the waist elastic member 25 to be attached, and therefore the flexibility and the stretchability of the waist member 20 are more likely to be maintained compared to the case of being fixed by the adhesive portions 70. In addition, the first sheet and the second sheet are joined over a wide region using a welding means such as ultrasonic welding, and the joining strength of the waist member 20 is more likely to be secured. This prevents tearing of the waist member 20 or peeling of the sheets even if the waist member 20 is pulled up when putting on the diaper 1. This makes it likely to ensure the strength of the waist member 20 while maintaining the flexibility thereof.

In addition, a plurality of the waist elastic members 25 (first elastic member E1) are provided spaced apart in the vertical direction, and between two waist elastic members 25 and 25 that are adjacent in the vertical direction, there are one or more welded portions 50 that are not in contact with the waist elastic members 25 (see FIG. 6). In other words, there are provided the welded portions 50 that does not have the function of attaching the first elastic member E1 by being sandwiched therebetween from above and below, and that is only for joining the skin-side sheet 21 (first sheet) and the non-skin-side sheets 22 and 23 (second sheet). In this way, by providing the welded portions 50 in portions that do not contribute to the attaching of the first elastic member E1, the joining strength of the first sheet and second sheet that constitute up the waist member 20 is further enhanced. Therefore, even if the waist member 20 is pulled when putting on the diaper 1, it can make it likely to further prevent the waist member 20 from being torn, and the like.

Additionally, on the back side of the waist member 20 in the front-back direction, the cover sheet 24 (third sheet) is provided so that the leg elastic members 27 (second elastic member E2) between the cover sheet 24 (third sheet) and the skin-side sheet 21 (first sheet). The non-skin-side sheet 23 (second sheet) and the cover sheet 24 (sheet 3) have a back overlapping portion LB that they overlap each other with respect to the vertical direction (see FIG. 3). Thus, in the back overlapping portion LB, at least three layers of the sheet member (21, 23, 24) are overlaid in the thickness direction, and this increases the strength compared to the rest of the waist member 20. This can make it easier to prevent the waist member 20 from tearing when the waist member 20 is pulled up at the time of putting on the diaper 1. In particular, the back side of the waist member 20 has a larger area than the front side, and accordingly is likely to come into contact with the wearer's skin when pulled up, creating greater resistance. Therefore, by increasing the strength with the back overlapping portion LB, it is possible to more easily prevent the waist member 20 from tearing, and the like.

In the back overlapping portion LB, the waist member 20 has a portion where the skin-side sheet 21 (first sheet), the non-skin-side sheet 23 (second sheet), and the cover sheet 24 (third sheet) are joined to one another. In FIG. 3, the skin-side sheet 21 (first sheet) and the non-skin-side sheet 23 (second sheet) are joined by the welded portions 50, and the non-skin-side sheet 23 (second sheet) and the cover sheet 24 (third sheet) are joined by the adhesive surface 75. And, the welded portion 50 and the adhesive surface 75 at least partially overlap with respect to the vertical direction. This makes the first to third sheets likely to integrate, making it possible to increase the strength of the back overlapping portion LB. This can make it more likely to prevent the waist member 20 from tearing and the like.

In addition, at least a part of the welded portion row 60 that is formed by a plurality of the welded portions 50, 50 .... located side-by-side spaced apart in the vertical direction is arranged so as to straddle the ends of the absorbent main body 10 in the lateral direction. In FIG. 6, the welded portion rows 60 are placed at positions that respectively overlap the two side ends 10es and 10es of the absorbent main body 10 with respect to the lateral direction. This increases the strength of the portions of the waist member 20 that overlap the two side ends 10es and 10es of the absorbent main body 10. The absorbent main body 10 and the waist member 20 are joined to each other by a hot-melt adhesive or the like. Therefore, when pulling the waist member 20 at the time of putting on the diaper 1, stress is concentrated at the boundary portion with the absorbent main body 10 (i.e., two side ends 10es and 10es of the absorbent body 10), causing a risk of making the waist member 20 more likely to tear. In contrast, in the present embodiment, the welded portion row 60 is formed so as to straddle the boundary portion (two side ends 10es) in the lateral direction, and this reinforces the strength of the waist member 20 at the boundary portion, making it possible to improve the durability of the waist member 20.

In addition, since the welded portion row 60 is formed extending along the vertical direction, the welded portion row 60 is arranged so as to straddle the upper end portion of the absorbent main body 10 in the vertical direction (see FIG. 6). Therefore, the strength of the waist member 20 is reinforced in the portion that overlaps the upper end of the absorbent main body 10, further improving the durability of the waist member 20.

In addition, in the upper end portion of the waist member 20, there are provided the fold-back portions 20Ff and 20Fb in which the skin-side sheet 21 (first sheet) and the non-skin-side sheets 22 and 23 (second sheets) are overlaid and folded from above to below (see FIG. 3 and the like). As a result, there is formed a portion in which four layers of sheet members are overlaid when the waist member 20 is viewed in the thickness direction. When putting on the diaper 1, the wearer or the like often holds the upper end portion of the waist member 20 with their hands and pulls it up. Therefore, when the strength increases locally due to increasing the number of the overlaid sheet members in the upper end portion of the waist member 20, it becomes more likely to prevent the waist member 20 from tearing or stretching excessively when holding and pulling up the upper end portion.

In the fold-back portions 20Ff and 20Fb, at least parts of the skin-side sheet 21 (first sheet) and the non-skin-side sheets 22 and 23 (second sheets) that are overlaid on each other are joined to each other by the welded portions 50 (see FIG. 3). Therefore, in the fold-back portions 20Ff and 20Fb, the first sheet and the second sheet are more likely to be integrated, resulting in greater strength. This prevents the two sheets of the sheet members from peeling off or one sheet of them from tearing when pulling the waist member 20, and this makes it possible to improve the wearability.

### Modified example

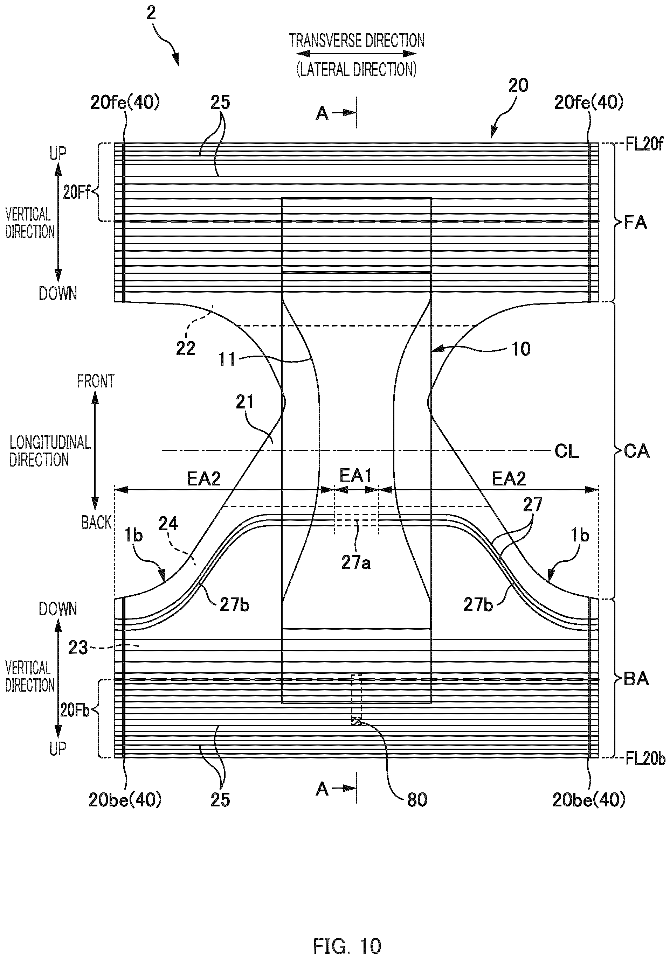

The diaper 1 according to the present embodiment may be modified as follows. FIG. 10 is a schematic plan view of a diaper 2 according to a modified example in the unfolded and stretched state. Regarding the configuration of the leg elastic members 27, the diaper 2 according to the modified example is partially different from the diaper 1. The rest of the configuration is basically the same as that of the diaper 1.

In each of the leg elastic members 27 of the diaper 2, a part of the portion that is located in the lateral central and that overlaps the absorbent core 11 is a low stretchy region, where the stretchability is lower than the remaining region. In FIG. 10, the portions of the leg elastic members 27 indicated by the dashed lines in the lateral center is the low stretchy region EA1, and the stretchability thereof is lower than that of the high stretchy regions EA2 indicated by the solid lines on both sides of it. In the low stretchy region EA1, each of the leg elastic members 27 is cut at multiple locations and is discontinuous in the lateral direction, decreasing the stretchability. In addition, the following configuration is acceptable: in the low stretchy region EA1, the adhesive portion 70 (see FIG. 8) is not provided; the leg elastic members 27 are cut at a certain point in the low stretchy region EA1; and the stretchability is made lower by contracting toward both lateral sides from the cut point (so-called cutback).

In the low stretchy region EA1, the stretchability of the leg elastic member 27 is weakened, and accordingly the contractive force in the lateral direction, which acts on the absorbent core 11, becomes smaller. Therefore, the width of the absorbent core 11 is prevented from narrowing when the diaper 2 is put on, making excrement less likely to leak.

In addition, in the diaper 2 of the modified example, in a region between the lateral one end and the lateral other end of the waist member 20, the total lateral length of the attachment means for attaching the first elastic member E1 (waist elastic member 25) to the waist member 20 (welded portions 50) is shorter than the total lateral length of the attachment means for attaching the second elastic member E2 (leg elastic member 27) to the waist member 20 (adhesive portions 70).

This makes the total length of the attachment means of the second elastic member E2 (leg elastic member 27) (adhesive portions 70) relatively longer, and as a result, the second elastic member E2 becomes more likely to be firmly fixed to the waist member 20, especially in the high stretchy regions EA2 (including the curved portions 27b) on both sides of the low stretchy region EA1. Therefore, when pulling the waist member 20 upward at the time of putting on the diaper 2, the force for pulling the waist member 20 is more likely to act on the absorbent core 11. With this configuration, in the diaper 2, it can make the absorbent core 11 more likely to fit to the wearer's crotch region while preventing the width of the absorbent core 11 from becoming narrow.

On the other hand, since the total length of the attachment means (welded portions 50) of first elastic member E1 is relatively short, it prevents the stiffness of the waist member 20 from becoming excessively high compared to the opposite case. This increases the flexibility of the waist member 20 and improves the fit, while making the texture softer during usage.

Furthermore, in the diaper 2, in a region between the lateral one end and the lateral other end of the waist member 20, the total lateral length of the attachment means for attaching the first elastic member E1 (waist elastic member 25) to the waist member 20 (welded portions 50) is shorter than the total lateral length of the attachment means for attaching the second elastic member E2 (leg elastic member 27) to the waist member 20 (adhesive portions 70) in the high stretchy regions EA2.

Since the total length of the attachment means of the second elastic member E2 (leg elastic member 27) (adhesive portions 70) in the high stretchy regions EA2 becomes relatively longer, the curved portion 27b becomes more likely to be firmly fixed to the waist member 20. Therefore, a force is more likely to transmit in the vertical direction (in a direction intersecting the lateral direction) via the curved portions 27b of the high stretchy regions EA2. As a result, when pulling the waist member 20 upward at the time of putting on the diaper 2, the force for pulling the waist member 20 is more likely to further act on the absorbent core 11. This can further increase the wearability of the absorbent core 11.

Also, in the diaper 2, the second elastic member E2 (leg elastic member 27) is attached to the waist member 20 throughout the lengthwise-direction range by the attachment means (adhesive portion 70) in which an adhesive such as a hot-melt adhesive is used. Therefore, the integrity of the waist member 20 and the leg elastic member 27 increase. As a result, the second elastic member E2 (leg elastic member 27) and the waist member 20 work together when putting on the diaper 2, and this can make it likely to pull up the absorbent core 11.

In addition, between the first sheet and the second sheet in the thickness direction, the first elastic member E1 (waist elastic member 25) is attached by being sandwiched between the pair of welded portion pairs 50s from above and below. Therefore, compared to the case of being fixed by the adhesive portions 70, the flexibility and the stretchability of the waist member 20 are more likely to be maintained, and the joining strength of the waist member 20 is more likely to be secured.

### Others

The above embodiment of the present disclosure is simply to facilitate understanding of the present disclosure and is not in any way to be construed as limiting the present disclosure. The present disclosure may variously be changed or altered without departing from its gist and encompass equivalents thereof.

In the above embodiment, as an example of the underpants-shaped absorbent article, there is described the diaper 1 in which the skin-side sheet 21 of the waist member 20 is configured in an integrated manner continuously from the one side (front side) to the other side (back side) in the longitudinal direction (see FIG. 3 and the like). But the skin-side sheet 21 may be configured as separate members on the front and back sides. For example, a so-called three-piece type underpants-shaped absorbent article may be acceptable which consists of three parts: a front exterior member (front waist portion), a back exterior member (back waist portion), and an absorbent main body.

In the waist member 20 of the diaper 1, a post-handling tape 80 may be provided at a predetermined position. The post-handling tape 80 is a substantially rectangular tape-like member (tape member) elongated in the vertical direction (longitudinal direction), and is made of a resin material such as polyethylene or polypropylene, for example. In the diaper 1, as shown in FIGS. 2 and 3, the post-handling tape 80 is provided on the non-skin-side surface of the back waist portion BA of the waist member 20 (the non-skin-side surface of the non-skin-side sheet 23). The post-handling tape 80 is fixed to the waist member 20 on the one side in the vertical direction and has a sticky portion on the other side in the vertical direction. An adhesive is applied to the sticky portion, and before the diaper 1 is used, a portion of the post-handling tape 80 is in a state of being folded with the sticky portion on the inside, thus preventing exposure of the sticky portion to the outside and protecting a sticky surface.

When discarding of the diaper 1 after use, the diaper 1 is rolled up in the vertical direction such that the absorbent main body 10 faces inward, and the folded post-handling tape 80 is pulled out to expose the sticky portion, and the sticky portion side is wrapped around the diaper 1. Accordingly, the diaper 1 can be held in the rolled state, and the diaper 1 can be discarded without allowing the leakage of excrement and the like adhering to the inside (absorbent main body 10) of the diaper 1.

In the diaper 1, at least some of the welded portions 50, which joins the skin-side sheet 21 (first sheet) and the non-skin-side sheet 23 (second sheet), have a portion that overlap the post-handling tape 80 with respect to the vertical direction and the lateral direction (see FIG. 3 and the like). Therefore, in the back waist portion BA of the waist member 20, the stiffness of the waist member 20 is higher in the portion where the post-handling tape 80 and the welded portion 50 overlap when viewed in the thickness direction compared to the non-overlapping portion. This prevents the waist member 20 from being excessively stretched or torn when pulling the waist member 20 at the time of putting on the diaper 1, and this can improve the wearability.

### REFERENCE SIGNS LIST

1 diaper (underpants-shaped absorbent article),
1a waist opening, 1b leg opening,
2 diaper (underpants-shaped absorbent article) (modified example),
   10 absorbent main body,
11 absorbent core, 11b core-wrapping sheet,
12 top sheet, 13 back sheet, 15 side sheet, 15t end portion,
20 waist member,
20Ff front fold-back portion, 20Fb back fold-back portion,
21 skin-side sheet, 22 non-skin-side sheet, 23 non-skin-side sheet, 24 cover sheet, 25 waist elastic member,
27 leg elastic member, 27a straight portion, 27b curved portion,
30 leak-proof wall portion,
35 leak-proof-wall elastic member,
40 side joining portion,
50s welded portion, 50s welded portion pair,
60 welded portion row,
70 adhesive portion, 75 adhesive surface,
80 post-handling tape,
E1 first elastic member, E2 second elastic member,
CL center position,
FA front waist portion, BA back waist portion, CA crotch portion,
FL20f front bending position, FL20b back bending position,
LB back overlapping portion

## Claims

1. An underpants-shaped absorbent article having a vertical direction, a lateral direction, and a front-back direction that intersect each other,
the underpants-shaped absorbent article comprising:
an absorbent main body that has a liquid-absorbent absorbent core;
a waist member; and
a first elastic member and a second elastic member on at least a one side of the waist member in the front-back direction,
the first elastic member being arranged at a position that does not overlap the absorbent core with respect to the vertical direction and being capable of stretching/contracting in the lateral direction,
the second elastic member being arranged at a position that overlaps the absorbent core with respect to the vertical direction and being capable of stretching/contracting in the lateral direction,
in a region where the waist member in the stretched state and the absorbent core overlap with respect to the lateral direction,
a total lateral length of an attachment means for attaching the first elastic member to the waist member
being shorter than
a total lateral length of an attachment means for attaching the second elastic member to the waist member.

2. The underpants-shaped absorbent article according to claim 1, wherein
a part of the second elastic member is arranged extending in a direction that intersects the lateral direction.

3. The underpants-shaped absorbent article according to claim 1 or 2, wherein
the second elastic member is attached to the waist member by the attachment means in which an adhesive is used.

4. The underpants-shaped absorbent article according to claim 3, wherein
the second elastic member is attached to the waist member in a region outside the absorbent core in the lateral direction by the attachment means in which the adhesive is used.

5. The underpants-shaped absorbent article according to claim 3, wherein
the underpants-shaped absorbent article has a pair of leg openings, and
the second elastic member is attached to the waist member in a region that extends along a contour of each of the leg openings, by the attachment means in which the adhesive is used.

6. The underpants-shaped absorbent article according to claim 3, wherein
the waist member has a first sheet and a second sheet that is overlaid on a non-skin side with respect to the first sheet,
the first sheet and the second sheet are joined to each other by a plurality of welded portions that are dispersed, and
the first elastic member is attached to the waist member by being sandwiched between two of the welded portions that are adjacent in the vertical direction.

7. The underpants-shaped absorbent article according to claim 6, wherein
the waist member has a plurality of the first elastic members spaced apart in the vertical direction, and
the waist member has the welded portion between two of the first elastic members that are adjacent in the vertical direction,
the welded portion not being contact with the two of the first elastic members.

8. The underpants-shaped absorbent article according to claim 6, wherein
the second elastic member is sandwiched between the first sheet and a third sheet that is overlaid on a non-skin side with respect to the first sheet, and
the second sheet and the third sheet have a portion where the second sheet and the third sheet overlap with respect to the vertical direction.

9. The underpants-shaped absorbent article according to claim 8, wherein
a portion where the first sheet and the second sheet are joined and a portion where the second sheet and the third sheet are joined partially overlap with respect to the vertical direction.

10. The underpants-shaped absorbent article according to claim 6, wherein
the underpants-shaped absorbent article has a welded portion row in which a plurality of the welded portions are located spaced apart in the vertical direction, and
at least a part of the welded portion row is arranged straddling an outer end of the absorbent main body in the lateral direction.

11. The underpants-shaped absorbent article according to claim 6, wherein
the underpants-shaped absorbent article has a fold-back portion in an upper end portion of the waist member,
the fold-back portion being a portion in which the first sheet and the second sheet that are overlaid are folded together from above to below in the vertical direction.

12. The underpants-shaped absorbent article according to claim 11, wherein
in the fold-back portion, at least parts of the first sheet and the second sheet are joined to each other by the welded portion.

13. The underpants-shaped absorbent article according to claim 6, wherein
the underpants-shaped absorbent article has a tape member that is arranged in a non-skin-side surface of the waist member, and
at least a part of the welded portion that joins the first sheet and the second sheet has a portion that overlaps the tape member with respect to the vertical direction and the lateral direction.

14. An underpants-shaped absorbent article having a vertical direction, a lateral direction, and a front-back direction that intersect each other,
the underpants-shaped absorbent article comprising:
an absorbent main body that has a liquid-absorbent absorbent core;
a waist member; and
a first elastic member and a second elastic member on at least a one side of the waist member in the front-back direction,
the first elastic member being arranged at a position that does not overlap the absorbent core with respect to the vertical direction and being capable of stretching/contracting in the lateral direction,
the second elastic member being arranged below the first elastic member in the vertical direction and being capable of stretching/contracting in the lateral direction,
the second elastic member having a curved portion that is curved inward from outside in the lateral direction and from above to below in the vertical direction,
in a region between a lateral one end and a lateral other end of the waist member in the lateral direction,
a total lateral length of an attachment means for attaching the first elastic member to the waist member
being shorter than
a total lateral length of an attachment means for attaching the second elastic member to the waist member.

15. The underpants-shaped absorbent article according to claim 14, wherein
the second elastic member has a low stretchy region in at least a part of a region that overlaps the absorbent core with respect to the lateral direction,
the low stretchy region having a stretchability that is lower than a remaining region, and
in a region outside the low stretchy region in the lateral direction,
a total length of the attachment means for attaching the first elastic member to the waist member
is shorter than
a total length of the attachment means for attaching the second elastic member to the waist member.

16. The underpants-shaped absorbent article according to claim 14 or 15, wherein
the second elastic member is attached to the waist member by the attachment means in which an adhesive is used.

17. The underpants-shaped absorbent article according to claim 16, wherein
the waist member has a first sheet and a second sheet that is overlaid on a non-skin side with respect to the first sheet,
the first sheet and the second sheet are joined to each other by a plurality of welded portions that are dispersed, and
the first elastic member is attached to the waist member by being sandwiched between two of the welded portions that are adjacent in the vertical direction.
